# EUROPÄISCHE PATENTSCHRIFT

(11) **EP 0 859 613 B1**
(45) Veröffentlichungstag und Bekanntmachung des Hinweises auf die Patenterteilung: **01.02.2006**
(21) Anmeldenummer: 96945822.3
(22) Anmeldetag: 23.10.1996
(51) Int. Cl.: A61K 31/44, A61P 37/04

(54) **VERWENDUNG VON FLUPIRTIN ZUR PROPHYLAXE UND THERAPIE VON ERKRANKUNGEN, DIE MIT EINER BEEINTRÄCHTIGUNG DES HÄMATOPOETISCHEN ZELLSYSTEMS EINHERGEHEN**
USE OF FLUPIRTIN FOR THE PREVENTION AND TREATMENT OF DISEASES WHICH ARE ASSOCIATED WITH DAMAGE TO THE HAEMOPOIETIC CELL SYSTEM
UTILISATION DE FLUPIRTINE POUR LA PROPHYLAXIE ET LE TRAITEMENT DE MALADIES ASSOCIEES A UNE ALTERATION DU SYSTEME CELLULAIRE HEMATOPOIETIQUE

(30) Priorität: 07.11.1995 DE 19541405
(43) Veröffentlichungstag der Anmeldung: 26.08.1998
(73) Patentinhaber: VIATRIS GmbH & Co. KG, 61352 Bad Homburg (DE)
(72) Erfinder: PERGANDE, Gabriela, D-63071 Offenbach (DE); MÜLLER, Werner, E., G., D-65203 Wiesbaden (DE)
(86) Internationale Anmeldenummer: PCT/DE1996/002009
(87) Internationale Veröffentlichungsnummer: WO 1997/017072

(56) Entgegenhaltungen:
- DE-A- 4 327 516
- CELL DEATH AND DIFFERENTIATION, Bd. 4, Nr. 1, Januar 1997, Seiten 51-56, XP000673261 W.E.G.MÜLLER ET AL.: "Flupirtine protects both neuronal cells and lymphocytes against induced apoptosis in vitro: Implications for treatment of AIDS patients"
- BRAIN RES., Bd. 667, Nr. 2, 1994, Seiten 291-294, XP000671785 N.N.OSBORNE ET AL.: "Immunohistochemical evidence for flupirtine acting as an antagonist on the N-methyl-D-aspartate and homocysteic acid-induced release of GABA in the rabbir retina"
- EUR.J.PHARMACOL., Bd. 317, Nr. 1, 12.Dezember 1996, Seiten 157-164, XP000673257 S.PEROVIC ET AL.: "Flupirtine increases the levels of glutathione and Bcl-2 in hNT (human Ntera/D1) neurons: mode of action of the drug-mediated anti-apoptotic effect"
- EUR.J.PHARMACOL., Bd. 288, Nr. 1, 15.Dezember 1994, Seiten 27-33, XP000673247 S.PEROVIC ET AL.: "The triaminopyridine flupirtine prevents cell death in rat cortical cells induced by N-Methyl-D-aspartate and gp120 of HIV-1"
- T W NEUROLOGIE PSYCHIATRY, Bd. 9, Nr. 10, Oktober 1995, Seite 606 XP000673251 B. GROOS: "Analgetikum Flupirtin schützt vor dem Zelltod"
- MOL.IMMUNOL., Bd. 28, Nr. 6, 1991, Seiten 641-654, XP000673253 J.M.VARGA ET AL.: "MECHANISM OF ALLERGIC CROSS-REACTIONS-I. MULTISPECIFIC BINDING OF LIGANDS TO A MOUSE MONOCLONAL ANTI-DNP IgE ANTIBODY"
- AIDS-FORSCHUNG, Bd. 9, Nr. 11, November 1994, Seite 603 XP000672112 W.E.G.MÜLLER ET AL.: "Flupirtin verhindert die durch NMDA und gp120 induzierte Apoptosis kortikaler Zellen der Ratte"
- SOCIETY FOR NEUROSCIENCE ABSTRACTS, Bd. 20, Nr. 1-2, 1994, Seite 451 XP000671787 W.E.G.MÜLLER ET AL.: "FLUPIRTINE PREVENTS APOPTOSIS INDUCED IN RAT CORTICAL CELLS BY NMDA AS WELL AS BY GP120 OF HIV-1"
- 11TH INT. CONF. AIDS, Bd. 11, Nr. 1, 7. - 12.Juli 1996, Seite 313 XP002031673 W.E.G.MÜLLER ET AL.: "FLUPIRTINE PROTECTS BOTH NEURONAL CELLS AND LYMPHOCYTES AGAINST INDUCED APOPTOSIS IN VITRO: IMPLICATIONS FOR TREATMENT OF AIDS PATIENTS"
- NEURODEGENERATION, Bd. 4, Nr. 4, Dezember 1995, Seiten 369-374, XP000673259 S.PEROVIC ET AL.: "Flupirtine Partially Prevents Neuronal Injury Induced by Prion Protein Fragment and Lead Acetate"
- PROG.MOL.SUBCELL.BIOL., Bd. 16, Nr. Apoptosis, 1996, Seiten 44-57, XP002031674 W.E.G.MÜLLER ET AL.: "Neurotoxicity in Rat Cortical Cells Caused by N-Methyl-D-Aspartate (NMDA) and gp120 of HIV-1: Induction and Pharmacological Intervention"
- ANTIVIRAL RESEARCH, Bd. 30, Nr. 1, 1996, Seite A38 XP000671786 WEG MÜLLER ET AL.: "FLUPIRTINE PARTIALLY PREVENTS NEURONAL INKURY INDUCED BY PRION PROTEIN FRAGMENT AND LEAD ACETATE"
- NEUROBIOLOGY OF AGING, Bd. 17, Nr. 4Suppl., 1996, Seite S152 XP000671886 W.E.G.MÜLLER ET AL.: "Protective Effect of the Drug Flupirtine on beta-Amyloid-induced Apoptosis in Primary Neuronal Cells in Vitro"
- SOCIETY FOR NEUROSCIENCE ABSTRACTS, Bd. 22, Nr. 1-3, 1996, Seite 2139 XP002031675 W.E.G.MÜLLER ET AL.: "FLUPIRTINE PROTECTS NEURONAL CELLS AGAINST INDUCED APOPTOSIS IN VITRO: IMPLICATIONS FOR THE TREATMENTOF ALZHEIMER'S DISEASE, cREUTZFELDT-jAKOB DISEASE AND AIDS PATIENTS"

## Beschreibung

Die Erfindung betrifft die Verwendung von Flupirtin oder dessen Salzen als Arzneimittel zur Behandlung von durch Medikamente (wie Cytostatike, Corticosteroide)-Giftstoff-, Strahlen- und durch andere Erkrankungen indurierter Neutropenie/Lymphozytopenie oder durch Medikamente oder infektiös (beispielweise HIV) induzierter Thrombozytopenie

Flupirtin ist ein bekanntes eingeführtes zentral wirksames, nicht-opiates Analgetikum.

Seine analgetischen Wirkungen entfaltet Flupirtin über andere Wirkmechanismen als die Opiat/Opioid-Analgetika (Nickel, B., Postgrad, Med. J. 63 (Suppl.3), 19 (1987); Szelenyi, I., Nickel, B., Borbe,: H. O., Brune ,K., Br. J. Pharmacol. 143,89 (1989)). In elektrophysiologischen Untersuchungen wurde gezeigt, daß Flupirtin sowohl auf der supraspinalen als auch auf der spinalen Ebene in das nozizeptive Geschehen einzugreifen vermag (Carisson, K. H., Jurna, I., Eur. J. Pharmakol. 143,89 (1987); Bleyer, H., Carlsson K. H., Erkel, H. J.,Jurna, I., Eur. J. Pharmacol. 151,259 (1988); Nickel, B., Aledter, A., Postgrad Med. J. 63 (Suppl.3) 41 (1987)).

Flupirtin wird auch bei akuten Schmerzzuständen, die durch Erkrankungen des Bewegungsapparates verursacht werden, zur Therapie verwendet.

Weiterhin wird Flupirtin erfolgreich bei Patienten mit degenerativen oder rheumatischen Erkrankungen eingesetzt.

Neben guten analgetischen Eigenschaften verfügt Flupirtin über muskelrelaxierende Eigenschaften, so daß Flupirtin auch für die Behandlung von Muskelverspannungen oder bei Erkrankungen, die auf Muskelverspannungen beruhen, eingesetzt werden kann(DE 40 22 442).

Desweiteren wurde bei Untersuchungen der muskelrelaxierenden Wirkung von Flupirtin an der Ratte gefunden, daß die Flupirtin-Wirkung sich durch die exitatorische Aminosäure N-Methyl-D-Aspartat (NMDA) hemmen läßt. Aufgrund dieser NMDA-antagonistischen Wirkung ist Flupirtin auch zur Behandlung von NMDA vermittelten ZNS-Erkrankungen, wie zum Beispiel zerebraler Ischämie, neurodegenerativer Erkrankungen, epileptischer Anfälle geeignet (DE 43 27 516).

Chemisch gesehen handelt es sich bei Flupirtin um 2-Amino-3-ethoxycarbonylamino-6-(p-fluor-benzylamino)-pyridin.

Die Synthese von Flupirtin und dessen pharmazeutisch verwendbaren Salzen wird in den Patenten DE 17 95 858, DE 31 33 519 und DE 34 16 609 beschrieben.

Es wurde nun überraschend gefunden, daß Flupirtin in der Lage ist, den apoptotischen Zelltod von Zellen des hämatopoetischen Zellsystems , beispielsweise der Lymphozyten, zu hemmen.

Damit eröffnet sich die Möglichkeit, Flupirtin zur Behandlung von durch Medikamente- (wie Cytostatike, Corticosteroide)-, Giftstoff-, Strahlen- und durch andere Erkrankungen indurierter Neutropenie/Lymphozytopenie oder durch Medikamente oder infektiös (beispielweise HIV) induzierter Thrombozytopenie, einzusetzen.

Besondere Bedeutung kommt dabei der Behandlung der oben genannten Krankheiten bei HIV-infizierten Patienten / AIDS-Patienten zu.

Anhand der pharmakologischen Untersuchungen soll die neue erfindungsgemäße Wirkung näher erläutert werden.

### Pharmakologische Untersuchungen

### Materialien

Flupirtin-Maleat [2-Amino-3-ethoxycarbonylamino-6-(p-fluorbenzylamino)-pyridin-maleat], zur Präparation von HIV-1-gp120 wurde der HIV-1 Virusstamm HTLV_{IIIB} eingesetzt (Popovic et al., 1984). Die erhaltene, isolierte gp120-Präparation war > 95% rein, wie durch Polyacrylamid-Gelelektrophorese nachgewiesen wurde (Müller et al., 1988).

### Antiretroviraler Versuchsansatz

Die Methodologie für den HIV-1-Ansatz wurde bereits früher beschrieben (Sarin et al., 1987). Zellen der menschlichen T-Lymphoblasten-Linie **CEM** (Einsaat-Konzentration: 1 x 10⁵ Zellen/ml) wurden in Medium suspendiert und mit dem HIV-1-Virus (Stamm HTLV_{IIIB}) infiziert. Hierzu wurde das 50-fache einer 50%-Zellkultur-Infektionsdosis [CCID₅₀] gewählt. Eine CCID₅₀ ist diejenige infektiöse Dosis bei der 50% der Zellen pro ml Zellsuspension infiziert werden. 1 ml-Kulturen wurden für 5 Tage inkubiert. Flupirtin wurde den Zellen in unterschiedlichen Konzentrationen 2 Stunden vor der Infektion zugesetzt. Die Anzahl der lebenden Zellen wurde unter Anwendung des 3-[4,5-Dimethylthiazol-2-yl]-2,5-diphenyltetrazolium-Bromid [**MTT**] kolorimetrischen Testsystems bestimmt (Scudiero et al., 1988). Die Auswertung geschah mit einem ELISA-Reader, wie früher publiziert (Müller et al., 1991). Nach der Inkubation machten die nichtinfizierten CEM-Zellen 2,16 und die infizierten Zellen 0,13 Verdoppelungsschritte durch.

### Blutproben

Proben menschlichen peripheren Blutes wurden (i) 12 HIV-1-infizierten homosexuellen Männern erhalten [mittleres Alter: 35 Jahre; Spanne: 22 - 43 Jahre] sowie (ii) 8 gesunden, HIV-1-seronegativen und ansonsten risikogleichen Männern der nahezu gleichen Altersgruppe (mittleres Alter: 30 Jahre; Spanne: 23 - 35 Jahre) entnommen. Die Patienten wurden entsprechend der Klassifikation des "Centers for Disease Control (CDC)" eingestuft (CDC, 1992). Die Untersuchungen mit HIV-1-seropositiven Patienten wurden mit Blutproben von asymptomatischen Virusträgern durchgeführt (CDC: A1, A2, mittlere CD4-Zellzahl 407/µl, Spanne: 209 - 698/µl).
Keiner der Patienten zeigte Anzeichen von Tumorerkrankungen, symptomatischen Infektionen oder war 10 Wochen vor der Blutentnahme einer immunsuppresiven Behandlung, z.B. mit Corticosteroiden, unterzogen worden.

### Zellpräparationen und deren Behandlung

Mononukleäre Zellen **[MNZ]** wurden aus heparinisiertem Blut durch Zentrifugation durch einen Ficoll-Hypaque-Dichtegradienten gewonnen und angereichert (Rossol et al., 1990). 0,5 x 10⁶ Zellen wurden in Kulturmedium in einem Endvolumen von 500 µl kultiviert; als Medium wurde **MEM**-Medium eingesetzt, das mit 10 mM HEPES-Puffer, 2,6% Natriumbicarbonat, 100 U/l Penicillin, 100 µg/ml Streptomycin, 10% fötalem Kälberserum [FKS] und 1 mM Glutamat angereichert wurde. Wo angegeben, wurden die Zellen sofort nach Isolierung mit der chemischen Substanz behandelt.

### Induktion der Apoptose mit reaktivem Sauerstoff

Reaktive Sauerstoff-Radikale wurden unter Anwendung des Hypoxanthin- [**HX**] und Xanthinoxidase [**XOD**] - Systemes hergestellt (Bruck et al., 1994).

MNZ wurden für 24 Stunden in einem Gesamtvolumen von 500 µl MEM Medium (wie oben beschrieben) suspendiert.
Diesem Ansatz wurden 0 bis 80 mU/ml XOD und 1 mM HX zugesetzt. Bei einer Konzentration von 80 mU/ml XOD (Sigma, München, FRG) unterlagen > 90% der Lymphozyten einem apoptotischen Zelltod.
Wenn nicht anders erwähnt, wurden für die nachfolgenden Studien 10 mU/ml des Enzyms XOD und 1 mM HX eingesetzt. Unter diesen Inkubationsbedingungen durchliefen etwa 50% der Zellen eine Apoptose. Es ist günstig, diesen Grad an Apoptose einzuhalten, wenn es zu testen gilt, ob eine bestimmte Verbindung einen potentiellen Inhibitor und/oder Stimulator von apoptotischem Zelltod darstellt Flupirtin wurde den Zellen 6 Stunden vor Zusatz des Sauerstoff-Radikal produzierenden Systemes zugegeben; die Inkubation wurde nach einem Tag abgeschlossen.

### Analyse der Zellen mittels Durchfluß-Zytometrie

Die Apoptose wurde in einem Durchfluß-Zytometer FACScan (Becton-Dickinson) mit einer Argon-Laser-Anregung bei einer Wellenlänge von 488 nm, entsprechend der publizierten Methode, gemessen (Schmid et al., 1994). Die Lymphozyten wurden durch optische Methoden getrennt und somit charakterisiert. Dies geschah durch Kombination einer Vorwärts-Lichtstreuung als Maß für die Größe der Zellen **[FSC]** mit einer rechwinkligen Streuung als Maß für die Oberflächen-Beschaffenheit [**SSC**]. Zur Anfärbung apoptotischer MNZ-Zellen wurden diese mit 20 µg/ml 7-Aminoactinomycin D [7-**AAD**] (Sigma), in **PBS** gelöst, für 20 Minuten bei 4°C unter Lichtausschluß inkubiert. Anschließend konnten die Zellen mit dem Durchfluß-Zytometer in der Färbelösung analysiert werden. Die rote Fluoreszenz, hervorgerufen durch 7-AAD wurde durch einen Filter der Wellenlänge 650 nm erfaßt.

7-AAD penetriert in apoptotische Zellen aufgrund einer Desintegration der Zellmembran und färbt im Anschluß daran DNA über einen Interkalationsvorgang an. Die Datenanalyse erfolgte über das LYSIS II Programm.
Die mittleren Kanalzahlen wurden umgerechnet auf mittlere Fluoreszenz-Intensität, Zellgröße und Oberflächenbeschaffenheit.

### In situ-Fluoreszenzmarkierung mittels des TdT Ansatzes sowie Oberflächenmarkierung

Um eine bestimmte Zell-Subpopulation, die einen apoptotischen Vorgang durchmacht, zu definieren, wurden die Zellen mit dem Terminal-Transferase-Systems [**TdT**] behandelt. Es wurde die Methode Gorczyca et al. (1993) mit geringen Modifikationen eingesetzt. Zellen in Suspension wurden zweimal in PBS (Gibco) gewaschen und anschließend in 1%igem Paraformaldehyd (Riedel de Haen) für 10 Min. bei 4°C fixiert. Danach wurden die Zellen zweimal in PBS [dem 5% AB-Serum und 0,5% bovines Serum-Albumin (**BSA**) zugesetzt waren] gewaschen. Weiterhin wurden die Zellen pelletiert und in 50 µl einer 2,5 mM Kobalt-Chlorid-Lösung, der 0,5 nM Biotin-16-dUTP und 25 U TdT zugesetzt wurde, resuspendiert. Gepuffert war dieser Ansatz mit 200 mM Kalium-Kakodylat, 25 mM Tris-HCl und 0,25 mg/ml BSA; die Inkubation erfolgte bei 37°C für 30 Min. entsprechend den Angaben des Herstellers (Boehringer Mannheim, Mannheim, FRG). Die Zellen wurden anschließend zweimal in PBS [5% AB-Serum und 0,5% BSA] gewaschen und in 100 µl einer gepufferter Farblösung, die 5 µg/ml Flurescein-Isothiocyanat [**FITC**]-markiertes Avidin [Zellanalys-Reinheit]enthielt, resuspendiert. Zur gleichzeitigen Oberflächen-Markierung wurden die Zellen noch zusätzlich mit 20 µg/ml Phycoerythrin-markiertem monoklonalen Anti-CD3-Antikörper (Becton-Dickinson) behandelt.
Die Fluoreszenz wurde mittels Durchfluß-Zytometrie, wie von Halliwell (1987) beschrieben, quantifiziert.

### Ergebnisse

### Behandlung von HIV-1 infizierten CEM-Zellen mit Flupirtin

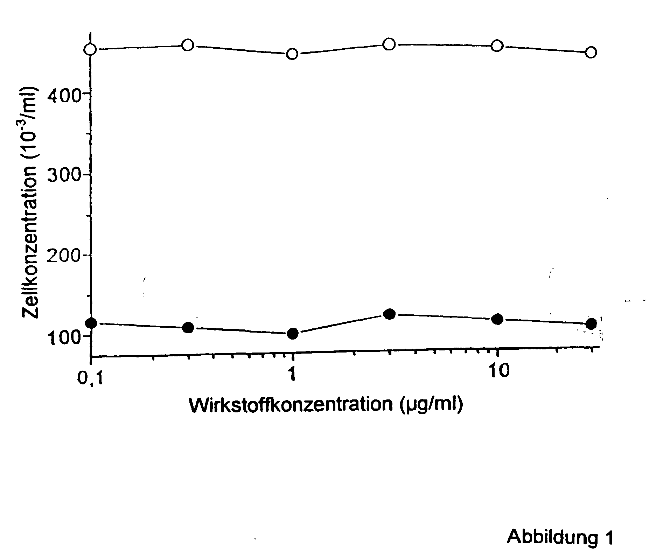

CEM-Zellen wurden mit Flupirtin für 2 Stunden vorbehandelt und verblieben anschließend nicht-infiziert oder wurden in einem Parallel-Ansatz mit HIV-1 infiziert.
Nach 5 Tagen wurden die Zellzahlen in den betreffenden Ansätzen bestimmt. Wie aus Abbildung 1 hervorgeht, veränderte sich die Konzentration der Zellen sowohl in den nicht-infizierten als auch den infizierten Kulturen in Abhängigkeit von der Behandlung mit Flupirtin nicht signifikant (P > 0,1).
Flupirtin wurde den Ansätzen in Endkonzentrationen zwischen 0,1 und 30 µg/ml zugegeben. Die Zell-Konzentration in den nicht-infizierten Ansätzen betrug 446.880 ± 31.020 Zellen/ml, in den HIV-1-infizierten Ansätzen 108.420 ± 6.710 Zellen/ml.
Diese Ergebnisse zeigen, daß Flupirtin nicht toxisch auf die Zellen wirkt, weiterhin aber auch keinen Einfluß auf eine HIV-1-Infektion in vitro unter den benutzten Inkubations-Bedingungen ausübt.

### Einstellung des Hypoxanthin/Xanthin-Oxidase-Systems

Die Konzentration an Xanthin-Oxidase [XOD] wurde so gewählt, das sich ein prozentualer Anteil an apoptotischen Zellen von etwa 50% einstellte.
Apoptotische Zellen zeigen eine Fluoreszenz-Intensität von > 10 arbiträren Kanalzahlen. Bei 0 mU an XOD lagen nur 6,6 ± 2,1% der Zellen oberhalb dieser "cut off"-Grenzlinie von > 10 arbiträren Kanalzahlen, waren also zu 6,6% apoptotisch. Bei zunehmender Konzentration an XOD nahm der prozentuale Anteil an apoptotischen Zellen zu und erreichte bei 80 mU XOD einen Wert von 93,2 ± 6,8% an apoptotischen Zellen. Bei 10 mU XOD durchliefen etwa 50% (genau 54,6 ± 6,1) der Zellen einen apoptotischen Prozess.

In Abwesenheit von XOD zeigen die Zellen ein FSC/SSC Verteilungsmuster wie folgt :
Zellgröße 349 ± 27 (arbiträre Kanalzahl) gegen eine Oberflächenbeschaffenheit von 112 ± 15. Bei einer Enzymkonzentration von 80 mU nahm die Größe der Zellen ab und erreichte einen Wert von 256 ± 22, während der Grad an Oberflächenbeschaffenheit auf 180 ± 29 zunahm. Solche Veränderungen zeigen eine charakteristische Apoptose, begründet auf morphologischen Veränderungen der Zellen, an. Bei 10 mU XOD zeigen die Zellen ein Verteilungsmuster, das etwa in der Mitte dieser vorher gezeigten Extremwerte lag. Wenn nicht anders aufgeführt, wurden 10 mU an Enzym eingesetzt.

### Effekt von Flupirtin auf die spontane Apoptoserate bei menschlichen Lymphozyten

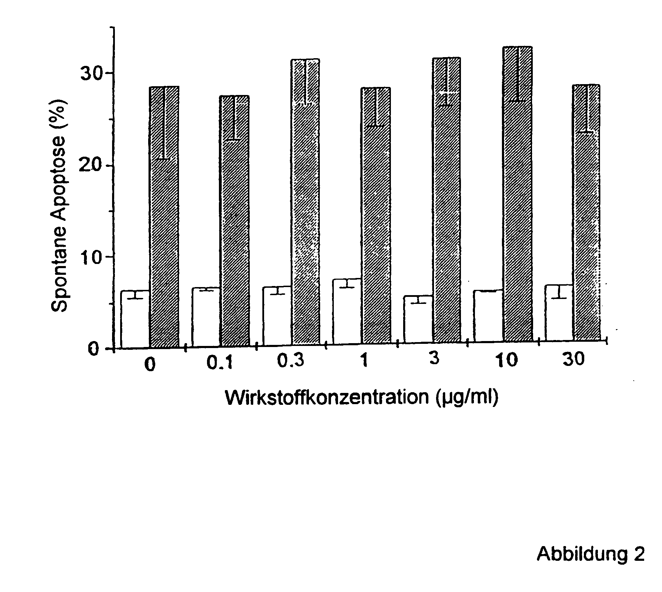

Periphere Blut-MNZ sowohl von gesunden Probanden als auch von HIV-1-infizierten Patienten wurden im Hinblick auf eine spontane Apoptose untersucht. Die Zellen wurden einen Tag nach Blutentnahme bei den Probanden analysiert.
Wie aus der Abbildung 2 ersichtlich ist, zeigen 6,32 ± 0,82% MNZ-Zellen von Kontrollgruppen nicht-infizierter Probanden nach dieserm Zeitraum Apoptose, während 28,42 ± 7,84% der Zellen von HIV-1-infizierten Patienten eine Apoptose durchliefen. MNZ beider Gruppen wurden für 24 Stunden mit Flupirtin in Konzentrationen von 0,1 bis 30 *µ*g/ml behandelt. Wie in der Abbildung 2 zusammengefasst, änderte sich die Rate der spontanen Apoptose von Lymphozyten unter Flupirtin-Einfluß nicht signifikant (P > 0.1). Dies trifft sowohl für Zellen von gesunden Probanden als auch von HIV-1-infizierten Patienten zu.

### Reduktion der induzierten Apoptose in mononukleären Zellen durch Flupirtin

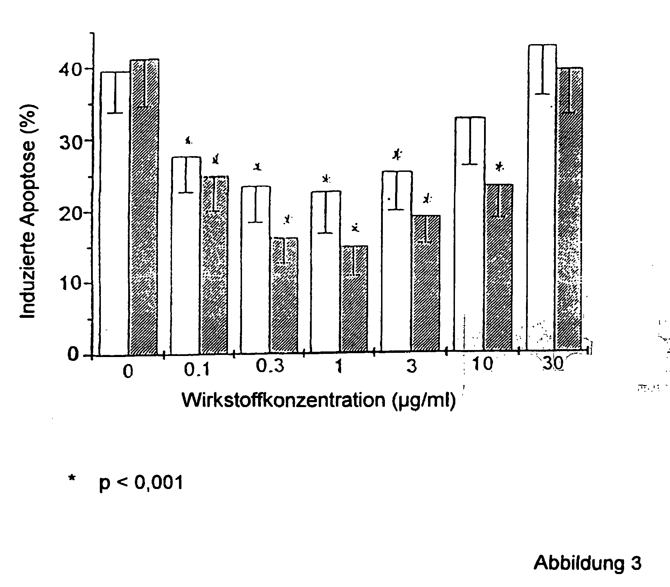

MNZ sowohl von gesunden Probanden als auch von HIV-1-infizierten Patienten wurden mit dem HX/XOD-System (Bildung von Sauerstoff-Radikalen), zur Induktion von Apoptose, behandelt. Flupirtin wurde den Zellansätzen 6 Stunden vor Induktor-Exposition in verschiedenen Konzentrationen zugegeben. Wie in Abbildung 3 gezeigt, bewirkte Flupirtin bei Konzentrationen zwischen 0,1 und 3,0 *µ*g/ml eine signifikante Reduktion des apoptotischen Zelltodes (P < 0,001). Bei einer Flupirtin-Konzentration von 10 µg/ml war der protektive Effekt nur noch in Assays mit Lymphozyten von infizierten Probanden signifikant. Bei Konzentrationen von 0,3 bis 3,0 µg/ml Flupirtin war der protektive Effekt des Arzneimittels am ausgeprägtesten und hemmte die induzierte Apoptose am stärksten.
Die Reduktion der induzierten Apoptose in den Ansätzen mit Lymphozyten von gesunden Probanden betrug etwa 40% und in solchen von HIV-1-infizierten Patienten etwa 60%.

Die Apoptose wurde in Lymphozyten auch über die Methode der DNA-Fragmentierung nachgewiesen. Nach Inkubation der Zellen mit dem HX/XOD-System wurde die DNA aus den Zellen extrahiert, im Agarose-Gel aufgetrennt nach der Größe und anschließend auf einen Blot übertragen.
Die DNA zerfiel in ein leiterartiges Muster aus Fragmenten von 180 Basen-Paaren und Vielfachen davon. Ein solches Fragmentierungsmuster ist charakteristisch für die DNA-Zerstörung, die bei apoptotischen Prozessen auftritt (Wyllie, 1980). Die Zellen, die mit 1 µg/ml Flupirtin behandelt wurden, zeigten im Gegensatz dazu keine DNA-Fragmentierung.
Repräsentative Dot-blots, die den Effekt von Flupirtin auf Zellen zeigen, die sowohl eine spontane als auch eine induzierte Apoptose durchmachen, liegen ebenfalls vor.

Zur Durchführung dieser Serie an Experimenten wurde in MNZ mittels des HX/20mU XOD-Systems Apoptose induziert. Unter diesen Bedingungen wurden - in Abwesenheit von Flupirtin - 64,2 ± 7,9% der Zellen apoptotisch.
Wenn diese Zellen jedoch für 24 Stunden mit Flupirtin behandelt wurden, nahm der Anteil an apoptotischen Zellen signifikant auf 18,3 ± 5,8% ab.

Diese Ergebnisse zeigen eindeutig, daß Flupirtin ein vielversprechendes Arzneimittel beispielsweise zur Behandlung des induzierten apoptotischen Zelltodes von Lymphozyten bei AIDS-Patienten ist.

In DE-OS 43 27 516 wird die neuroprotektive Wirkung von Flupirtin beschrieben, beispielsweise bei cerebraler Ischämie, Morbus Alzheimer, Morbus Parkinson, infektiös induzierten Neurodegenerationserkrankungen wie die AIDS-Enzephalopathie oder Jakob-Creutzfeld-Erkrankung.

Die Indikation AIDS-Enzephalopathie mit fortschreitender Demenz stellt eine neurologische Komplikation der AIDS-Erkrankung dar. Als Ursache für das Entstehen der AIDS-Enzephalopathie wird unter anderem die HIV(gp 120) - induzierte Freisetzung von NMDA-agonistisch wirkenden Neurotoxinen wie Chinolinsäure aus infizierten Makrophagen angesehen, die letztendlich zu einem Absterben der Nervenzellen führt.

AIDS-Enzephalopathie und AIDS-Erkrankung (acquired immunodeficiency syndrome) sind verschiedene Organsysteme betreffende Erkrankungen. Bei der ersten Erkrankung handelt es sich, wie schon dargelegt, um eine neurologische Komplikation der AIDS-Erkrankung (Apoptose von Nervenzellen), während die zweite Erkrankung eine "erworbene Immuninsuffizienz" darstellt, die auf einer Störung des zellulären Immunsystems mit ausgeprägter Verminderung der T-Helfer-Zellen beruht(Apoptose eines Bestandteiles des hämatopoetischen Zellsystems).

Die Wirkung des Flupirtins, welches eine Hemmung der induzierten Apoptose von Zellen des hämatopoetischen Zellsystems, beispielsweise von Lymphozyten verursacht, war auch umso überraschender , da die Existenz von NMDA-Rezeptoren außerhalb des Zentralnervensystems bisher nicht nachgewiesen wurde. Demzufolge war es auch für einen Fachmann nicht möglich, von einem Schutz der Nervenzellen durch Flupirtin auf einen Schutz der Zellen des hämatopoetischen Zellsystems zu schließen.

Als Erkrankungen, die prophylaktisch und therapeutisch aufgrund der erfindungsgemäßen Wirksamkeit des Flupirtins behandelbar sind, werden genannt:
- Medikamenten(wie Cytostatika, Corticosteroide)-, Giftstoff-, Strahlen- und durch andere Erkrankungen induzierte Neutropenie/Lymphozytopenie
- Medikamenten- oder infektiös (beispielsweise HIV) induzierte Thrombozytopenie.

Flupirtin kann zur Prophylaxe und Therapie in bekannter Weise in folgenden Darreichungsformen verabreicht werden:

Tabletten, Filmtabletten, Hartgelatinekapseln, Weichgelatinekapseln, Pellets, Granulate, Dragees, Suppositorien, Mikrokapseln, wäßrige oder ölige Suspensionen, ölige Lösungen, Injektionslösungen zur intramuskulären Verabreichung, Injektionslösungen zur intravenösen Verabreichung.

Geeignete Salze für die Herstellung des Arzneimittels sind alle physiologisch verträglichen Salze des Flupirtins. Beispielsweise kommen das Hydrochlorid, Maleat, Sulfat und Gluconat von Flupirtin in Frage.

Die Gehalte an Flupirtin in den erfindungsgemäßen Arzneimitteln betragen 0,1 mg-3000 mg, vorzugsweise 10 mg-500 mg. Die genannten Einzeldosen des Arzneimittels können 1 - 5 mal, vorzugsweise 1 - 3 mal täglich verabreicht werden.

Die Dosierungsangaben beziehen sich immer auf Flupirtin als Base. Werden Salze des Flupirtins eingesetzt, so ist entsprechend dem Molgewicht umzurechnen.

Die pharmazeutische und galenische Handhabung der erfindungsgemäßen Verbindungen erfolgt nach den üblichen Standardmethoden. Beispielsweise wird Flupirtin und die Träger- und/oder Hilfsstoffe durch Rühren oder Homogenisieren gut vermischt, wobei im allgemeinen bei Temperaturen zwischen 20 und 80°C, vorzugsweise 20 bis 50°C, gearbeitet wird.

### Beschreibung der Abbildungen

### Abbildung 1

Inkubation von nicht-infizierten **[O]** und HIV-1-infizierten CEM Zellen [●] mit unterschiedlichen Konzentrationen an Flupirtin. Die Mittelwerte von 10 Parallel-Experimenten sind angegeben; die Standardabweichungen waren nicht höher als 10%.

### Abbildung 2

Effekt von Flupirtin auf die spontane Apoptose von Lymphozyten gewonnen von nicht-infizierten Probanden [offene Balken] und HIV-1-infizierten Patienten [diagonalgestreifte Balken]. Nach einem Tag wurden die Zellen mittels Durchflußzytometrie analysiert. Lymphozyten von 12 infizierten Patienten und 8 nicht-infizierten Probanden wurden untersucht; die Mittelwerte und die Standardabweichungen sind angegeben.

### Abbildung 3

Reduktion des induzierten apoptotischen Absterbens von Lymphozyten nach Behandlung mit Flupirtin. MNZ von nicht-infizierten Probanden [offene Balken] und HIV-1-infizierten Patienten [diagonal-gestreifte Balken] wurden mittels des HX/XOD-Systems zum apoptotischen Absterben gebracht. Flupirtin wurde den Zellen 6 Std. vor dem Sauerstoffradikal-generierenden System zugegeben. Einen Tag später wurden die Zellen mittels Durchflußzytometrie analysiert. Die Werte für die Gesamt-Apoptose wurden ermittelt; davon wurden die Werte für die Spontan-Apoptose subtrahiert. Somit geben die dargestellten Werte das Ausmaß an induzierter Apoptose an. Lymphozyten von 12 infizierten Patienten und 8 nicht-infizierten Probanden wurden untersucht; die Mittelwerte und die Standardabweichungen sind angegeben.

## Patentansprüche

1. Verwendung des Wirkstoffes Flupirtin oder dessen pharmazeutisch verwendbarer Salze zur Herstellung eines Arzneimittels zur Behandlung von durch Medikamente, wie Cytostatika,-Corticosteroide-, Giftstoff-, Strahlen- und durch andere Erkrankungen indurierter Neutropenie/Lymphozytopenie oder durch Medikamente oder infektiös, beispielsweise HIV indurierter Thrombozytopenie.

2. Verwendung gemäß Anspruch 1 **dadurch gekennzeichnet, dass** Flupirtin oder dessen Salze für die entsprechenden Anwendungsformen mit pharmazeutisch geeigneten Träger- und/oder Hilfsstoffen kombiniert werden.

## Claims

1. Use of the active compound flupirtine or its pharmaceutically usable salts for preparing a medicament for the treatment of neutropenia/lymphocytopenia induced by drugs, such as cytostatics and corticosteroids, toxins, radiation or other diseases or thrombocytopenia induced by drugs or infections, for example HIV.

2. Use according to Claim 1, **characterized in that** flupirtine or its salts are combined with pharmaceutically suitable excipients and/or auxiliaries to form appropriate administration forms.

## Revendications

1. Utilisation du principe actif flupirtine ou de ses sels utilisables d'un point de vue pharmaceutique pour préparer un médicament destiné au traitement de la neutropénie/lymphocytopénie induite par des médicaments, tels que des cytostatiques, des corticostéroïdes, par des poisons, des rayonnements, et par d'autres maladies, ou d'une thrombocytopénie induite par des médicaments ou d'origine infectieuse, par exemple due au VIH.

2. Utilisation selon la revendication 1, **caractérisée en ce que** la flupirtine ou ses sels sont, pour des formes posologiques appropriées, combinés à des excipients et/ou des adjuvants acceptables d'un point de vue pharmaceutique.
